# EUROPEAN PATENT SPECIFICATION

(11) **EP 3 041 539 B1**
(45) Date of publication and mention of the grant of the patent: **03.04.2019**
(21) Application number: 14761306.1
(22) Date of filing: 01.09.2014
(51) Int. Cl.: A61M 5/24

(54) **DRUG DELIVERY DEVICE COMPRISING A CAP SENSOR AND A RADIATION SENSOR**
MEDIKAMENTENVERABREICHUNGSGERÄT MIT EINEM KAPPENSENSOR UND EINEM STRAHLUNGSSENSOR
DISPOSITIF D'ADMINISTRATION DE MEDICAMENTS AVEC UN DETECTEUR DE BOUCHON ET UN DETECTEUR DE RADIATION

(30) Priority: 03.09.2013 EP 13182793
(43) Date of publication of application: 13.07.2016
(73) Proprietor: Sanofi-Aventis Deutschland GmbH, 65929 Frankfurt am Main (DE)
(72) Inventor: EGGERT, Ilona, 65926 Frankfurt am Main (DE); CASPERS, Michael, 65926 Frankfurt am Main (DE)
(74) Representative: Derry, Paul Stefan
(86) International application number: PCT/EP2014/068492
(87) International publication number: WO 2015/032715

(56) References cited:
- EP-A1- 1 640 029
- WO-A1-97/33638
- WO-A1-2010/100213

## Description

The present patent application inter-alia relates to an apparatus for ejecting a fluid having a housing comprising a cartridge retainer configured to hold a fluid containing cartridge and further comprising a cap connection part for removably attaching thereto a cap. The apparatus may in particular be a medical device.

The medical device can be an injector, for example a hand-held injector, especially a pen-type injector, that is an injector of the kind that provides for administration by injection of medicinal products from one or more multidose cartridges. In particular, the present invention relates to such injectors where a user may set the dose.

The drug agents may be contained in two or more multiple dose reservoirs, containers or packages, each containing independent (single drug compound) or pre-mixed (co-formulated multiple drug compounds) drug agents.

Certain disease states require treatment using one or more different medicaments. Some drug compounds need to be delivered in a specific relationship with each other in order to deliver the optimum therapeutic dose. The present patent application is of particular benefit where combination therapy is desirable, but not possible in a single formulation for reasons such as, but not limited to, stability, compromised therapeutic performance and toxicology.

For example, in some cases it may be beneficial to treat a diabetic with a long acting insulin (also may be referred to as the first or primary medicament) along with a glucagon-like peptide-1 such as GLP-1 or GLP-1 analog (also may be referred to as the second drug or secondary medicament).

Accordingly, there exists a need to provide devices for the delivery of two or more medicaments in a single injection or delivery step that is simple for the user to perform without complicated physical manipulations of the drug delivery device. The proposed drug delivery device provides separate storage containers or cartridge retainers for two or more active drug agents. These active drug agents are then combined and/or delivered to the patient during a single delivery procedure. These active agents may be administered together in a combined dose or alternatively, these active agents may be combined in a sequential manner, one after the other.

The drug delivery device also allows for the opportunity of varying the quantity of the medicaments. For example, one fluid quantity can be varied by changing the properties of the injection device (e.g., setting a user variable dose or changing the device's "fixed" dose). The second medicament quantity can be changed by manufacturing a variety of secondary drug containing packages with each variant containing a different volume and/or concentration of the second active agent.

The drug delivery device may have a single dispense interface. This interface may be configured for fluid communication with a primary reservoir and with a secondary reservoir of medicament containing at least one drug agent. The drug dispense interface can be a type of outlet that allows the two or more medicaments to exit the system and be delivered to the patient.

The combination of compounds from separate reservoirs can be delivered to the body via a double-ended needle assembly. This provides a combination drug injection system that, from a user's perspective, achieves drug delivery in a manner that closely matches the currently available injection devices that use standard needle assemblies. One possible delivery procedure may involve the following steps:
1. Attach a dispense interface to a distal end of the electro-mechanical injection device. The dispense interface comprises a first and a second proximal needle. The first and second needles pierce a first reservoir containing a primary compound and a second reservoir containing a secondary compound, respectively.
2. Attach a dose dispenser, such as a double-ended needle assembly, to a distal end of the dispense interface. In this manner, a proximal end of the needle assembly is in fluidic communication with both the primary compound and secondary compound.
3. Dial up/set a desired dose of the primary compound from the injection device, for example, via a graphical user interface (GUI).
4. After the user sets the dose of the primary compound, the micro-processor controlled control unit may determine or compute a dose of the secondary compound and preferably may determine or compute this second dose based on a previously stored therapeutic dose profile. It is this computed combination of medicaments that will then be injected by the user. The therapeutic dose profile may be user selectable. Alternatively, the user can dial or set a desired dose of the secondary compound.
5. Optionally, after the second dose has been set, the device may be placed in an armed condition. The optional armed condition may be achieved by pressing and/or holding an "OK" or an "Arm" button on a control panel. The armed condition may be provided for a predefined period of time during which the device can be used to dispense the combined dose.
6. Then, the user will insert or apply the distal end of the dose dispenser (e.g. a double ended needle assembly) into the desired injection site. The dose of the combination of the primary compound and the secondary compound (and potentially a third medicament) is administered by activating an injection user interface (e.g. an injection button).

Both medicaments may be delivered via one injection needle or dose dispenser and in one injection step. This offers a convenient benefit to the user in terms of reduced user steps compared to administering two separate injections.

There is a general desire to improve handling and user safety of medical devices. In particular, there is a desire to improve user safety of medicament delivery devices, in order to prevent for example administration of degenerated drugs to a patient, since degenerated drugs may have less or no medical effect resulting in a medicament underdosage, or they even may be harmful for the patient.

In the following, the term "light" means the visible light spectrum (380 nm to 780 nm) as well as the near-visible spectra such as ultraviolet (UV) light and infrared (IR) light. UV light is light with a wavelength in the range of 10 nm to 380 nm, and IR light is light with a wavelength in the range of 780 nm to 3000 nm.

Drug degeneration may occur due to ageing of the drug, but also due to excessive light radiation, as some drugs get instable or may decay when exposed to light, and especially to UV light. Drugs are often stored within cartridges, which provide a certain UV light protection. However, these cartridges are often transparent to allow visual inspection of the drug and of the filling state and therefore only provide insufficient light protection. When not in use, the cartridges are therefore typically stored in a dark package or inside a fridge or in another dark environment.

When drug containing cartridges are inserted into injection devices, additional light protection is also provided by the cartridge holder of the device. Since the cartridge holders are often transparent to allow the cartridge to be visually inspected from the outside, injection devices also comprise a light protecting cap to be attached to the device when the device is currently not used.

In order to perform an injection with such an injection device, the user however has to remove the protecting cap so that the drug may be exposed to light. This usually does not cause a problem if the drug is only exposed to light for a short length of time while the user is performing the injection. However, sometimes the user forgets to replace the cap after using the device, so that the drugs remain unprotected from light and may degenerate over time.

WO 2010/100213 A1 discloses a drug delivery device comprising an indicator means capable of revealing usage related information of the drug delivery device, the indicator means being movable with respect to the housing of the drug delivery device.

WO 97/33638 A1 discloses an injection device which has signal generators which are connected to operative elements of the device and which give off signals which represent the operative condition of the device.

EP 1640029 A1 discloses a drug delivery device capable of working in at least two different modes which includes a dose injection mode and a set-up mode. The drug delivery device automatically changes from the dose injection mode to the set-up mode and vice versa in accordance with the detection of a cover by a cover detection switch.

In light of the aforementioned, the invention inter-alia faces the technical problem of providing an apparatus, especially a medicament delivery device, which provides for better light protection of a fluid, especially of a drug.

According to the present invention, there is provided an apparatus according to claim 1, a system according to claim 13, and a method according to claim 15.

The apparatus may be a delivery device, especially a drug delivery device such as a medical device configured to eject a drug agent (e.g. a dose of a medicament) such as an infusion device or an injection device, for instance an insulin injection pen. Injection devices may be used either by medical personnel or by patients themselves. As an example, type-1 and type-2 diabetes may be treated by patients themselves by injection of insulin doses, for example once or several times per day.

In particular, the apparatus may be a medical device configured to deliver (e.g. eject) at least two drug agents from separate cartridges situated in two separate retainers.

Alternatively, the apparatus may for instance be configured to deliver (e.g. eject) a two-component adhesive from separate cartridges comprising a first component of the two-component adhesive (e.g. a binder) and a second component of the two-component adhesive (e.g. a hardener), respectively.

The housing comprises a cap connection part for removably attaching thereto a cap. For example, the cap may cover a small end portion of the housing, but it may also cover a substantial portion of the housing. In particular the cap may be designed as a cover. The connection part is designed accordingly to receive a corresponding cap. When attached to the cap connection part, the cap covers the drug container which contains the drug. Thus, the drug is completely blocked from light, including UV light.

The control unit is configured to cause a control step to be performed once the quantity exceeds a predefined threshold value. A control step is understood to mean a step, in which the apparatus is controlled to perform a certain action. For example, the control step may comprise the actions of setting the apparatus into a particular status or actuating a certain component of the apparatus such as the user interface to display a message, to generate an alarm tone or the like.

The control unit is configured to measure a quantity being a measure for the radiation load on a cartridge in the cartridge retainer. Being a measure for the radiation load on a cartridge in the cartridge retainer is understood to mean that the radiation load on a cartridge in the cartridge retainer can in principle be determined or estimated as a function of this quantity. For example, the quantity may be a function of the amount of radiation a cartridge in the retainer is actually exposed to. To this end, the quantity may be a function of a radiation signal from a radiation sensor provided within the apparatus. Alternatively, the quantity may also be a function of the length of time, during which the cartridge is possibly exposed to a radiation, in particular because the cap is not properly attached to the cap connection part. From such a quantity, the radiation load on a cartridge in the cartridge retainer may in principle be estimated by assuming a typical average radiation.

It is noted that the control unit does not necessarily need to actually determine or estimate the radiation load on a cartridge in the cartridge retainer as a function of the quantity. However, a value of the quantity may for example be selected as the predefined threshold value if the radiation load determined or estimated as a function of this value exceeds a certain radiation load threshold.

The apparatus described above allows controlling whether the fluid in the cartridge has been exposed to a radiation such as light radiation, especially UV light radiation, of a too large amount or for a too long length of time, so that there is a significant risk that substances in the fluid, such as active pharmaceutical ingredients, may have degenerated.

The object described above is further at least in part solved by a system comprising an apparatus as described above and a cap configured to be removably attached to the connection part of the apparatus.

The object described above is further at least in part solved by a method for controlling such an apparatus or such a system, respectively, comprising the following steps: measuring a quantity being a measure for the radiation load on a cartridge in the cartridge retainer; and performing a control step once the quantity exceeds a predefined threshold value.

A number of embodiments of the apparatus, of the system and of the method will be described in the following. Although these embodiments are described in particular with reference to the apparatus, they are not limited to the apparatus, but also apply to the system comprising such an apparatus and to the method for controlling such an apparatus or system accordingly.

According to an embodiment of the apparatus the control unit is configured to measure a radiation time length, the radiation time length being the length of a continuous time period during which a set of conditions is fulfilled, the set of conditions at least comprising the condition that the cap is not attached to the connection part, and wherein the control unit is configured to cause a control step to be performed once that radiation time length exceeds a predefined threshold time length. According to this embodiment the radiation time length is the quantity being a measure for the radiation load on a cartridge in the cartridge retainer. The radiation time length indicates the time length during which a cartridge in the cartridge retainer is likely to be exposed to a radiation, in particular to light, especially to UV light. The radiation time length is therefore a reliable and easy determinable quantity for the control unit to control the apparatus for performing a control step.

The control unit is configured to measure a radiation time length, which is the length of a continuous time period during which a set of conditions is fulfilled. The set of conditions at least comprises the condition that a cap is not attached to the connection part. This may be the only condition of the set of conditions. Alternatively further conditions may be comprised by the set of conditions, for example the condition that a cartridge is situated within the cartridge retainer.

According to a corresponding embodiment of the method, the method comprises the following steps: measuring a radiation time length, the radiation time length being the length of a continuous time period during which a set of conditions is fulfilled, the set of conditions at least comprising the condition that the cap is not attached to the connection part; and performing a control step once the radiation time length exceeds a predefined threshold time length.

According to a further embodiment of the apparatus, the apparatus comprises a radiation sensor, preferably located inside the cartridge holder, the radiation sensor being configured to measure the intensity of a radiation, preferably of light, in particular of UV light, wherein the control unit is configured to measure the amount of radiation employing the radiation sensor, and wherein the control unit is configured to cause a control step to be performed once the amount of radiation exceed a predefined threshold amount of radiation. According to this embodiment the amount of radiation is the quantity being a measure for the radiation load on a cartridge in the cartridge retainer. The amount of radiation may be determined by the control unit by integrating the radiation measured by the radiation sensor over time, in particular over a time period, so that the amount of radiation represents the accumulated radiation exposure over time. When the amount of radiation exceeds a predefined threshold amount of radiation, the control step is caused to be performed. The sensor may be located inside the cartridge holder or outside. It may also be implemented in or on the cartridge label of a cartridge, so that the apparatus comprises the sensor once the cartridge is inserted into the cartridge retainer. Being determined by means of a radiation sensor, the amount of radiation is a very precise quantity for the control unit to control the apparatus for performing a control step.

The control unit may also be configured to measure a plurality of quantities being a measure for the radiation load on a cartridge in the cartridge retainer and to cause a control step to be performed once one of the plurality of quantities exceeds a respective predefined threshold value. For example, the radiation time length and the amount of radiation may be measured by the control unit at the same time in order to improve redundancy.

According to an embodiment of the apparatus, the apparatus comprises a sensor configured to determine whether a cartridge is inside the device. The control unit may further be configured to reset (e.g. set to zero) the quantity being a measure for the radiation load on a cartridge in the cartridge retainer, in particular the radiation time length or the amount of radiation, once a cartridge is inserted into the device, so that for each new cartridge the measurement of the radiation time length or the radiation amount is started again.

According to another embodiment of the apparatus, the apparatus may comprise a colour sensor for determining the colour of a colour-changing part of a cartridge within the cartridge retainer, for example a part of the cartridge label. The control unit may furthermore be configured to cause a control step to be performed once the colour sensor detects a predefined colour or colour change. According to this embodiment the colour or the colour change measured by means of the colour sensor is the quantity being a measure for the radiation load on a cartridge in the cartridge retainer. For example, a cartridge used with the apparatus may comprise a label, a part of which changing its colour as a function of light exposure. The colour change of the label then represents the accumulated light exposure of the label (and therefore of the medicament within the cartridge) over time.

The housing is configured such that a cartridge in the cartridge retainer is protected from a radiation by the cap when the cap is attached to the cap connection part. This ensures that radiation protection or the cartridge and of a fluid therein is ensured if the cap is attached to the apparatus. For example, the housing may provide a partial radiation protection, in particular light radiation protection, for a cartridge in the retainer, while a part of the cartridge is only protected by the cap, i.e. when the cap is attached to the cap connection part. According to a further embodiment the apparatus further comprises a user interface for outputting messages to a user. The user interface may for example comprise one or more of the following: a graphical user interface such as an LCD / TFT display, a group of light emitting diodes, a speaker for emitting a sound, a vibration alarm, an illuminatable (e.g. blinking) button, or the like. Such a user interface allows outputting a message to the user, for example to remind him of replacing the cap. In a particular embodiment, the control step comprises outputting of a warning message by means of the user interface. This allows sending the user a warning message if the radiation time length exceeds the threshold time length, i.e. if there is a risk that the fluid in the cartridge is or will be degenerated if the cap is not attached to the cap connection part in time. For example, the control unit may cause outputting of an alarm such as an alarm tone, a vibration alarm, a blinking display or a blinking dose button to indicate that the cap has to be replaced. In particular, the control unit may be configured such that the alarm only stops if the cap is replaced to the apparatus. The control unit may also be configured to output graphical information such as a symbol, preferably without any text, on a display if the control step is performed before the user has set the language of the apparatus.

In addition, the apparatus may comprise a sensor configured to determine whether a cartridge is inside the cartridge retainer and the control unit may further be configured to only send out an alarm if a cartridge is inside the cartridge retainer.

According to a further embodiment of the apparatus the control step comprises setting the apparatus into a status, in which ejection of a fluid is only allowed after replacement of the cartridge in the cartridge retainer. To this end, the apparatus, in particular the cartridge retainer or the housing, may for example comprise a sensor to detect whether a cartridge is present in the retainer or not. The control unit may then determine by evaluating the signal from this sensor whether the cartridge has been replaced.

The control unit may set the apparatus into a status, in which ejection of a fluid is disallowed until a cartridge replacement has been performed and/or detected. Setting the apparatus into a particular status may for example comprise setting a status variable in a storage of the control unit to a predefined value, wherein the control unit is configured such that at least one of the possible further control steps of the apparatus are a function of the status variable. For example, value "0" in the status variable may indicate regular operation, "1" may indicate a standby-status and "2" may indicate a status, in which ejection of a fluid is disallowed.

Alternatively, setting the apparatus into a particular status may for example comprise jumping to a particular position in the program flow of a computer program run by the control unit, such as a particular subroutine or a particular command sequence.

By setting the apparatus into a status in which ejection of a fluid is disallowed, the apparatus prevents that a possibly degenerated fluid is ejected from the apparatus. Ejection of a fluid is only permitted again, if the fluid cartridge has been replaced, i.e. if there is fresh and non-degenerated fluid available for ejection. In case of a medicament delivery device, this prevents administration of possibly degenerated drugs if there is a risk that the drug has been excessively exposed to light radiation.

The term "ejection of a fluid" is understood to mean the action of ejecting a fluid from the cartridge, which action is usually controlled by the control unit. In case of a medicament delivery device, ejection of a fluid is understood to mean in particular delivery of a medicament from a medicament cartridge to, for example, a patient.

According to a further embodiment of the apparatus the control unit is configured to cause a first control step to be performed once the radiation time length exceeds a predefined first threshold time length and wherein the control unit is configured to cause a second control step to be performed once the radiation time length exceeds a predefined second threshold time length, the second threshold time length being longer than the first threshold time length. In particular, the first control step may be different from the second control step. This embodiment allows a more differentiated control of the apparatus as a function of the radiation time and the according risk of degeneration.

According to a further embodiment the apparatus comprises a user interface for outputting messages to a user, the first control step comprises outputting of a warning message by means of the user interface and the second control step comprises setting the apparatus into a status, in which ejection of a fluid is only allowed after replacement of the cartridge in the cartridge retainer. This embodiment provides two levels of escalation depending on the radiation time length. After a first radiation time length, the user may for example be warned of possible drug degeneration if the cap is not replaced to the housing and, after a second radiation time length when drug degeneration is more likely, the control unit may prevent further drug ejection from the apparatus. For example, the first control step may comprise outputting a symbol or text on a display, outputting a sound by means of a speaker and/or outputting a light signal, for example by causing an illuminatable button such as the dose button to blink.

According to a further embodiment of the apparatus the set of conditions comprises the condition that no user activatable button is operated. Apparatuses usually comprise one or more buttons that may be operated by a user to control the apparatus. For example, a drug delivery device may comprise a user activatable ejection button to initiate ejection of a medicament. User activatable buttons may for example be physical buttons such as push or rotary buttons or virtual buttons displayed on a touch-screen terminal. Operation of buttons indicates that a user is currently using the device. Since a user is usually aware of the cap not being attached to the apparatus which he is currently using, it is usually undesirable that a control step caused by exceeding radiation time length is performed while the user still uses the apparatus. The embodiment described here ensures that a control step is only performed if a user has not operated a button for a longer time, i.e. when it is likely that he does not currently use the apparatus but forgot to replace the cap. This increases user friendliness of the apparatus.

According to a further embodiment of the apparatus the set of conditions comprises the condition that the apparatus is in a stand-by status. For example, the control unit may set the apparatus into a stand-by status if no user activatable button has been operated for a predefined length of time. The stand-by status may in particular be an energy-saving status of the device, in which for example a display of the apparatus is turned off. A stand-by status usually indicates that the apparatus is no longer used by the user. Thus, the apparatus being in a stand-by status is a good indicator that the cap should be attached to the housing to prevent the cartridge or the fluid therein from radiation. It is another advantage of this embodiment that during the control step the user may not only be reminded of replacing the cap but also to completely turn off the apparatus and thus to save energy.

According to a further embodiment of the apparatus the sensor is a reed sensor or a hall sensor. A reed sensor is an electrical switch comprising a pair of ferromagnetic reeds forming a pair of contacts. The contacts may be normally open and closing when a magnetic field is present or vice versa. A hall sensor is a sensor making use of the Hall effect and usually having an output voltage depending on the present magnetic field strength. For example, the apparatus may comprise the reed or hall sensor and the cap may comprise a magnet at a corresponding position. When the cap is attached to the connection part, the reed or hall sensor detects the presence of the magnetic field of the magnet and thus indicates that the cap is attached. Reed sensors and Hall sensors are reliable, long-life and low- or no-maintenance sensors and therefore are particularly suitable for the apparatus. Moreover, the cap only has to be equipped with a magnet to cooperate with the sensor.

According to further embodiment the apparatus is a medical device, in particular a medicament ejection device. Some drugs get instable if exposed to light. Furthermore it may be dangerous to administer drugs degenerated by light since they may have less beneficial or even harmful effects on the patient. Therefore the apparatus described above is in particular advantageous for medical devices.

According to a further embodiment the apparatus is hand-held. A hand-held apparatus is often an apparatus that is operated by laymen such as patients instead of medically trained personnel. The safety and usability increase achieved with the apparatus described above is therefore in particular advantageous for hand-held apparatuses.

According to an embodiment of the system, the material, the wall thickness and/or the colour of the cap is selected such that the light transmittance, especially the transmittance of UV light, of the cap (i.e. the fraction of incident light passing through the cap) is less than 1 %, preferably less than 0.1 %, in particular less than 0.01 %. Thus the cartridge may be protected from light radiation when the cap is attached to the cap connection part. For example, the cap may be made of a material having a dark colour or containing UV absorbing additives. The cap may also have a UV protection coating.

These as well as other advantages of various aspects of the present invention will become apparent to those of ordinary skill in the art by reading the following detailed description, with appropriate reference to the accompanying drawings, in which:
- Fig. 1: illustrates a perspective view of a delivery device with an end cap of the device removed;
- Fig. 2: illustrates a perspective view of the delivery device distal end showing the cartridge;
- Fig. 3: illustrates a perspective view of the delivery device illustrated in Fig. 1 or 2 with one cartridge retainer in an open position;
- Fig. 4: illustrates a dispense interface and a dose dispenser that may be removably mounted on a distal end of the delivery device illustrated in Fig. 1;
- Fig. 5: illustrates the dispense interface and the dose dispenser illustrated in Fig. 4 mounted on a distal end of the delivery device illustrated in Fig. 1;
- Fig. 6: illustrates one arrangement of a needle assembly that may be mounted on a distal end of the delivery device;
- Fig. 7: illustrates a perspective view of the dispense interface illustrated in Fig. 4;
- Fig. 8: illustrates another perspective view of the dispense interface illustrated in Fig. 4;
- Fig. 9: illustrates a cross-sectional view of the dispense interface illustrated in Fig. 4;
- Fig. 10: illustrates an exploded view of the dispense interface illustrated in Fig. 4;
- Fig. 11: illustrates a cross-sectional view of the dispense interface and needle assembly mounted onto a drug delivery device, such as the device illustrated in Fig. 1;
- Fig. 12: illustrates a perspective view of the delivery device comprising a sensor for detecting whether the cap is attached to the device; and
- Fig. 13: shows a flow chart shows a control sequence for controlling the apparatus when the cap is not attached to the housing.

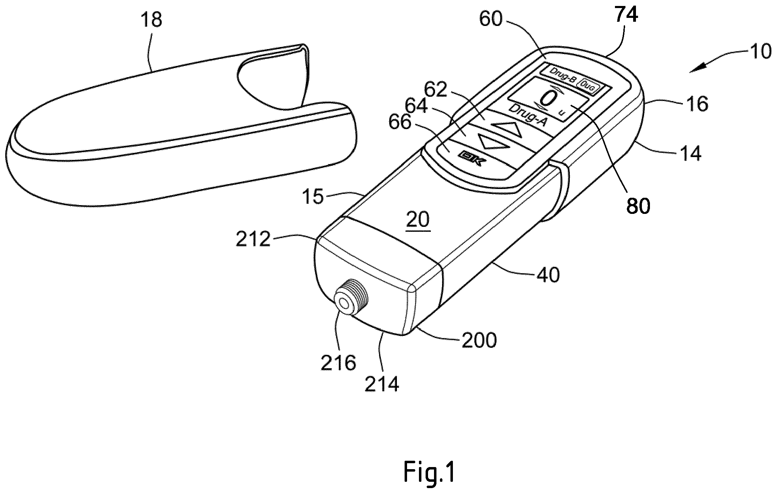

The drug delivery device illustrated in Fig. 1 comprises a main body 14 that extends from a proximal end 16 to a distal end 15. At the distal end 15, a removable end cap or cover 18 is provided. This end cap 18 and the distal end 15 of the main body 14 work together to provide a snap fit or form fit connection so that once the cover 18 is slid onto the distal end 15 of the main body 14, this frictional fit between the cap and the main body outer surface 20 prevents the cover from inadvertently falling off the main body.

The main body 14 contains a micro-processor control unit, an electro-mechanical drive train, and at least two medicament reservoirs. When the end cap or cover 18 is removed from the device 10 (as illustrated in Fig. 1), a dispense interface 200 is mounted to the distal end 15 of the main body 14, and a dose dispenser (e.g., a needle assembly) is attached to the interface. The drug delivery device 10 can be used to administer a computed dose of a second medicament (secondary drug compound) and a variable dose of a first medicament (primary drug compound) through a single needle assembly, such as a double ended needle assembly.

The drive train may exert a pressure on the bung of each cartridge, respectively, in order to expel the doses of the first and second medicaments. For example, a piston rod may push the bung of a cartridge forward a pre-determined amount for a single dose of medicament. When the cartridge is empty, the piston rod is retracted completely inside the main body 14, so that the empty cartridge can be removed and a new cartridge can be inserted.

A control panel region 60 is provided near the proximal end of the main body 14. Preferably, this control panel region 60 comprises a digital display 80 along with a plurality of human interface elements that can be manipulated by a user to set and inject a combined dose. In this arrangement, the control panel region comprises a first dose setting button 62, a second dose setting button 64 and a third button 66 designated with the symbol "OK." In addition, along the most proximal end of the main body, an injection button 74 is also provided (not visible in the perspective view of Fig. 1). The user interface of the drug delivery device may comprise additional buttons, such as a "menu" button, a "back" button, or a "light" button to switch on an illumination of the display.

The cartridge holder 40 can be removably attached to the main body 14 and may contain at least two cartridge retainers 50 and 52. Each retainer is configured so as to contain one medicament reservoir, such as a glass cartridge. Preferably, each cartridge contains a different medicament.

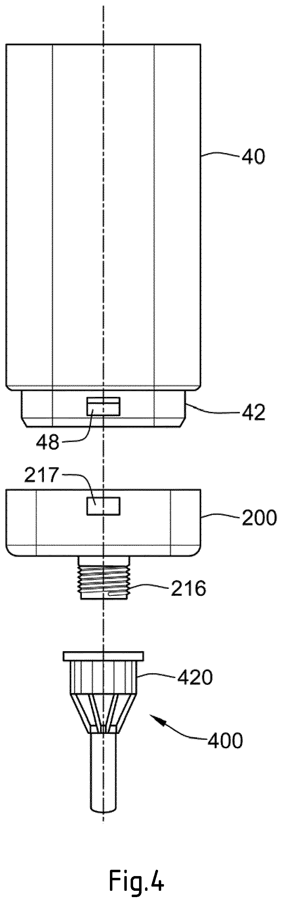

In addition, at the distal end of the cartridge holder 40, the drug delivery device illustrated in Fig. 1 includes a dispense interface 200. As will be described in relation to Fig. 4, in one arrangement, this dispense interface 200 includes a main outer body 212 that is removably attached to a distal end 42 of the cartridge housing 40. As can be seen in Fig. 1, a distal end 214 of the dispense interface 200 preferably comprises a needle hub 216. This needle hub 216 may be configured so as to allow a dose dispenser, such as a conventional pen type injection needle assembly, to be removably mounted to the drug delivery device 10.

Once the device is turned on, the digital display 80 shown in Fig. 1 illuminates and provides the user certain device information, preferably information relating to the medicaments contained within the cartridge holder 40. For example, the user is provided with certain information relating to both the primary medicament (Drug A) and the secondary medicament (Drug B).

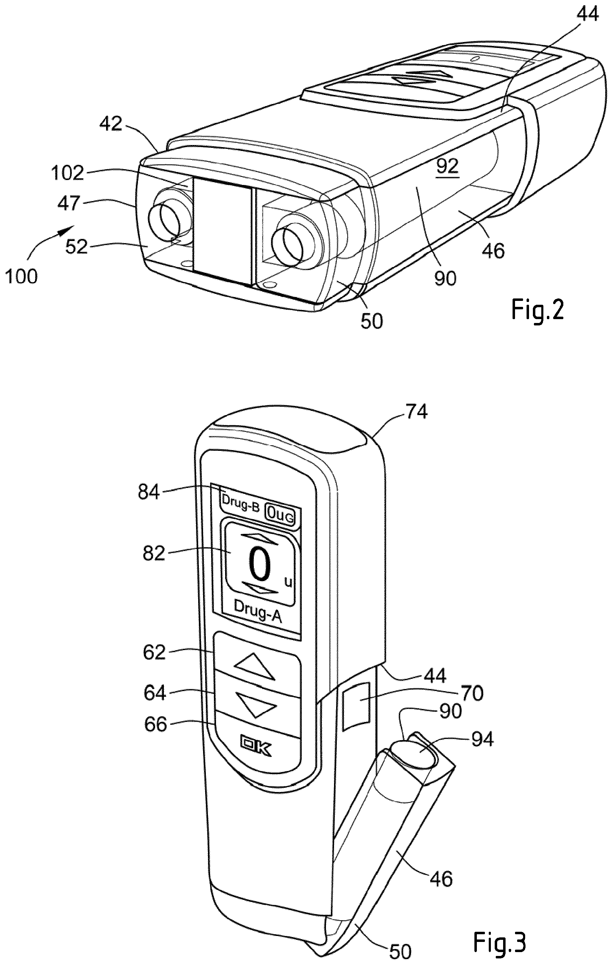

As shown in Fig. 3, the first and second cartridge retainers 50, 52 may be hinged cartridge retainers. These hinged retainers allow user access to the cartridges. Fig. 3 illustrates a perspective view of the cartridge holder 40 illustrated in Fig. 1 with the first hinged cartridge retainer 50 in an open position. Fig. 3 illustrates how a user might access the first cartridge 90 by opening up the first retainer 50 and thereby having access to the first cartridge 90.

As mentioned above when discussing Fig. 1, a dispense interface 200 can be coupled to the distal end of the cartridge holder 40. Fig. 4 illustrates a flat view of the dispense interface 200 unconnected to the distal end of the cartridge holder 40. A dose dispenser or needle assembly 400 that may be used with the interface 200 is also illustrated and is provided in a protective outer cap 420.

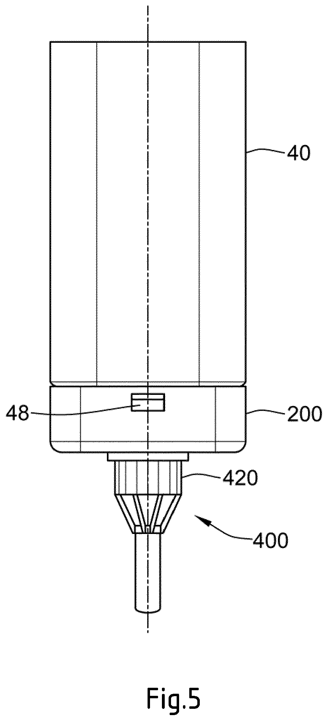

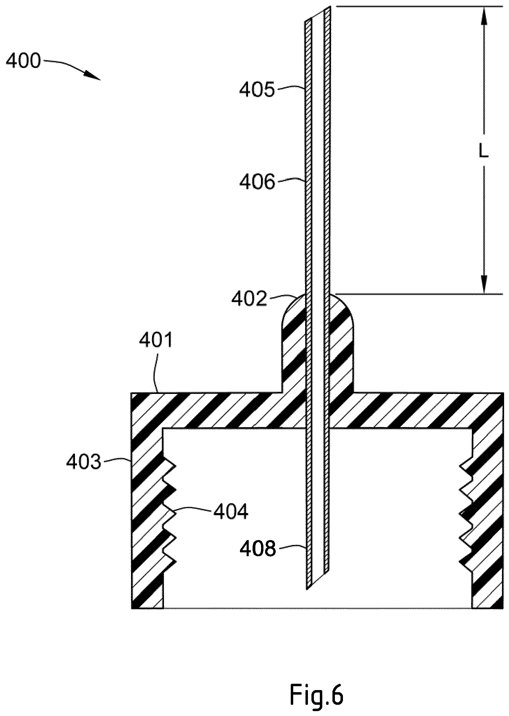

In Fig. 5, the dispense interface 200 illustrated in Fig. 4 is shown coupled to the cartridge holder 40. The axial attachment means 48 between the dispense interface 200 and the cartridge holder 40 can be any known axial attachment means to those skilled in the art, including snap locks, snap fits, snap rings, keyed slots, and combinations of such connections. The connection or attachment between the dispense interface and the cartridge holder may also contain additional features (not shown), such as connectors, stops, splines, ribs, grooves, pips, clips and the like design features, that ensure that specific hubs are attachable only to matching drug delivery devices. Such additional features would prevent the insertion of a non-appropriate secondary cartridge to a non-matching injection device. Fig. 5 also illustrates the needle assembly 400 and protective cover 420 coupled to the distal end of the dispense interface 200 that may be screwed onto the needle hub of the interface 200. Fig. 6 illustrates a cross sectional view of the double ended needle assembly 400 mounted on the dispense interface 200 in Fig. 5.

The needle assembly 400 illustrated in Fig. 6 comprises a double ended needle 406 and a hub 401. The double ended needle or cannula 406 is fixedly mounted in a needle hub 401. This needle hub 401 comprises a circular disk shaped element which has along its periphery a circumferential depending sleeve 403. Along an inner wall of this hub member 401, a thread 404 is provided. This thread 404 allows the needle hub 401 to be screwed onto the dispense interface 200 which, in one preferred arrangement, is provided with a corresponding outer thread along a distal hub. At a center portion of the hub element 401 there is provided a protrusion 402. This protrusion 402 projects from the hub in an opposite direction of the sleeve member. A double ended needle 406 is mounted centrally through the protrusion 402 and the needle hub 401. This double ended needle 406 is mounted such that a first or distal piercing end 405 of the double ended needle forms an injecting part for piercing an injection site (e.g., the skin of a user).

Similarly, a second or proximal piercing end 408 of the needle assembly 400 protrudes from an opposite side of the circular disc so that it is concentrically surrounded by the sleeve 403. In one needle assembly arrangement, the second or proximal piercing end 408 may be shorter than the sleeve 403 so that this sleeve to some extent protects the pointed end of the back sleeve. The needle cover cap 420 illustrated in Fig. 4 and 5 provides a form fit around the outer surface 403 of the hub 401.

Referring now to Fig. 4 to 11, one preferred arrangement of this interface 200 will now be discussed. In this one preferred arrangement, this interface 200 comprises:
a. a main outer body 210,
b. an first inner body 220,
c. a second inner body 230,
d. a first piercing needle 240,
e. a second piercing needle 250,
f. a valve seal 260, and
g. a septum 270.

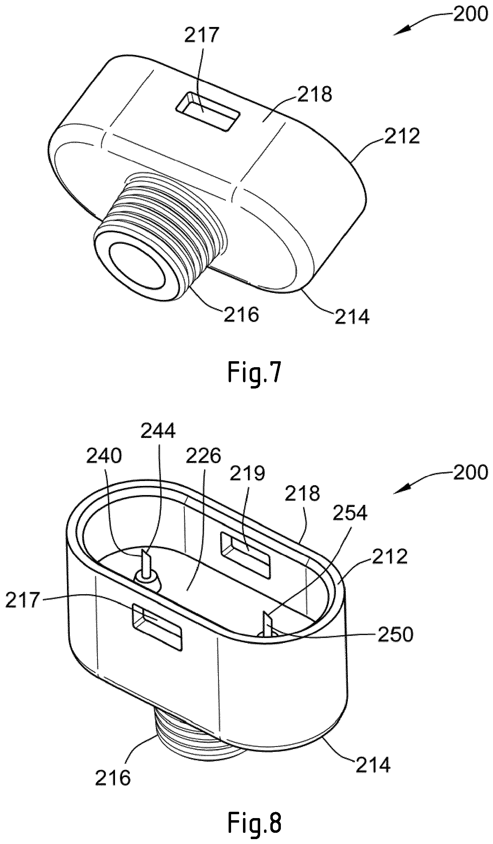

The main outer body 210 comprises a main body proximal end 212 and a main body distal end 214. At the proximal end 212 of the outer body 210, a connecting member is configured so as to allow the dispense interface 200 to be attached to the distal end of the cartridge holder 40. Preferably, the connecting member is configured so as to allow the dispense interface 200 to be removably connected the cartridge holder 40. In one preferred interface arrangement, the proximal end of the interface 200 is configured with an upwardly extending wall 218 having at least one recess. For example, as may be seen from Fig. 8, the upwardly extending wall 218 comprises at least a first recess 217 and a second recess 219.

Preferably, the first and the second recesses 217, 219 are positioned within this main outer body wall so as to cooperate with an outwardly protruding member located near the distal end of the cartridge housing 40 of the drug delivery device 10. For example, this outwardly protruding member 48 of the cartridge housing may be seen in Fig. 4 and 5. A second similar protruding member is provided on the opposite side of the cartridge housing. As such, when the interface 200 is axially slid over the distal end of the cartridge housing 40, the outwardly protruding members will cooperate with the first and second recess 217, 219 to form an interference fit, form fit, or snap lock. Alternatively, and as those of skill in the art will recognize, any other similar connection mechanism that allows for the dispense interface and the cartridge housing 40 to be axially coupled could be used as well.

The main outer body 210 and the distal end of the cartridge holder 40 act to form an axially engaging snap lock or snap fit arrangement that could be axially slid onto the distal end of the cartridge housing. In one alternative arrangement, the dispense interface 200 may be provided with a coding feature so as to prevent inadvertent dispense interface cross use. That is, the inner body of the hub could be geometrically configured so as to prevent an inadvertent cross use of one or more dispense interfaces.

A mounting hub is provided at a distal end of the main outer body 210 of the dispense interface 200. Such a mounting hub can be configured to be releasably connected to a needle assembly. As just one example, this connecting means 216 may comprise an outer thread that engages an inner thread provided along an inner wall surface of a needle hub of a needle assembly, such as the needle assembly 400 illustrated in Fig. 6. Alternative releasable connectors may also be provided such as a snap lock, a snap lock released through threads, a bayonet lock, a form fit, or other similar connection arrangements.

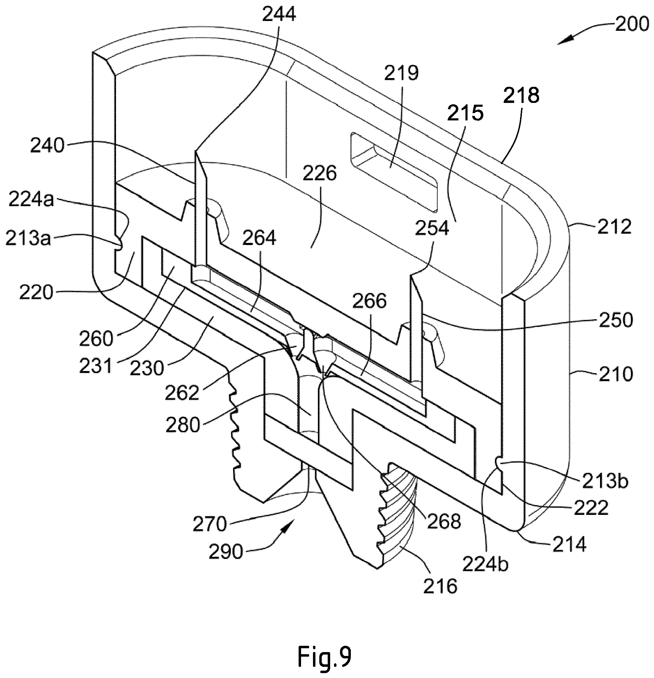

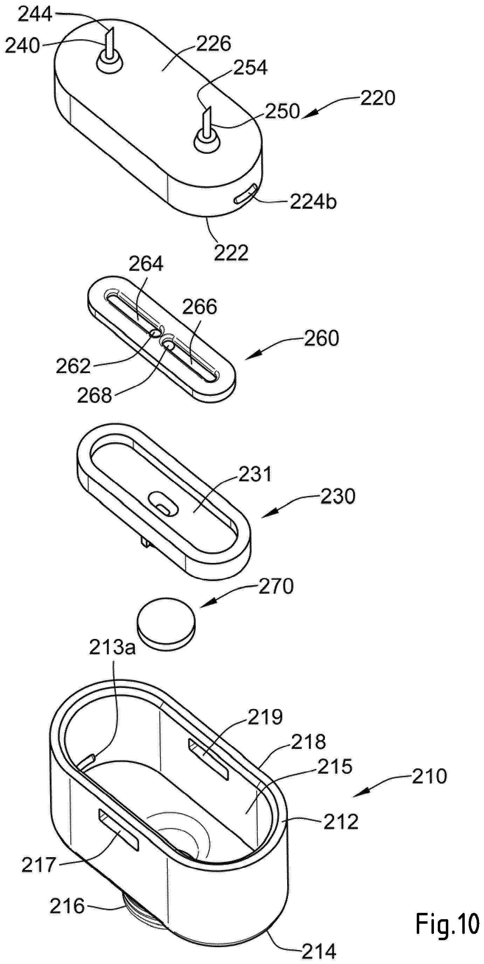

The dispense interface 200 further comprises a first inner body 220. Certain details of this inner body are illustrated in Fig. 8-11. Preferably, this first inner body 220 is coupled to an inner surface 215 of the extending wall 218 of the main outer body 210. More preferably, this first inner body 220 is coupled by way of a rib and groove form fit arrangement to an inner surface of the outer body 210. For example, as can be seen from Fig. 9, the extending wall 218 of the main outer body 210 is provided with a first rib 213a and a second rib 213b. This first rib 213a is also illustrated in Fig. 10. These ribs 213a and 213b are positioned along the inner surface 215 of the wall 218 of the outer body 210 and create a form fit or snap lock engagement with cooperating grooves 224a and 224b of the first inner body 220. In a preferred arrangement, these cooperating grooves 224a and 224b are provided along an outer surface 222 of the first inner body 220.

In addition, as can be seen in Fig. 8-10, a proximal surface 226 near the proximal end of the first inner body 220 may be configured with at least a first proximally positioned piercing needle 240 comprising a proximal piercing end portion 244. Similarly, the first inner body 220 is configured with a second proximally positioned piercing needle 250 comprising a proximally piercing end portion 254. Both the first and second needles 240, 250 are rigidly mounted on the proximal surface 226 of the first inner body 220.

Preferably, this dispense interface 200 further comprises a valve arrangement. Such a valve arrangement could be constructed so as to prevent cross contamination of the first and second medicaments contained in the first and second reservoirs, respectively. A preferred valve arrangement may also be configured so as to prevent back flow and cross contamination of the first and second medicaments.

In one preferred system, dispense interface 200 includes a valve arrangement in the form of a valve seal 260. Such a valve seal 260 may be provided within a cavity 231 defined by the second inner body 230, so as to form a holding chamber 280. Preferably, cavity 231 resides along an upper surface of the second inner body 230. This valve seal comprises an upper surface that defines both a first fluid groove 264 and second fluid groove 266. For example, Fig. 9 illustrates the position of the valve seal 260, seated between the first inner body 220 and the second inner body 230. During an injection step, this seal valve 260 helps to prevent the primary medicament in the first pathway from migrating to the secondary medicament in the second pathway, while also preventing the secondary medicament in the second pathway from migrating to the primary medicament in the first pathway. Preferably, this seal valve 260 comprises a first non-return valve 262 and a second non-return valve 268. As such, the first non-return valve 262 prevents fluid transferring along the first fluid pathway 264, for example a groove in the seal valve 260, from returning back into this pathway 264. Similarly, the second non-return valve 268 prevents fluid transferring along the second fluid pathway 266 from returning back into this pathway 266.

Together, the first and second grooves 264, 266 converge towards the non-return valves 262 and 268 respectively, to then provide for an output fluid path or a holding chamber 280. This holding chamber 280 is defined by an inner chamber defined by a distal end of the second inner body both the first and the second non return valves 262, 268 along with a pierceable septum 270. As illustrated, this pierceable septum 270 is positioned between a distal end portion of the second inner body 230 and an inner surface defined by the needle hub of the main outer body 210.

The holding chamber 280 terminates at an outlet port of the interface 200. This outlet port 290 is preferably centrally located in the needle hub of the interface 200 and assists in maintaining the pierceable seal 270 in a stationary position. As such, when a double ended needle assembly is attached to the needle hub of the interface (such as the double ended needle illustrated in Fig. 6), the output fluid path allows both medicaments to be in fluid communication with the attached needle assembly.

The hub interface 200 further comprises a second inner body 230. As can be seen from Fig. 9, this second inner body 230 has an upper surface that defines a recess, and the valve seal 260 is positioned within this recess. Therefore, when the interface 200 is assembled as shown in Fig. 9, the second inner body 230 will be positioned between a distal end of the outer body 210 and the first inner body 220. Together, second inner body 230 and the main outer body hold the septum 270 in place. The distal end of the inner body 230 may also form a cavity or holding chamber that can be configured to be fluid communication with both the first groove 264 and the second groove 266 of the valve seal.

Axially sliding the main outer body 210 over the distal end of the drug delivery device attaches the dispense interface 200 to the multi-use device. In this manner, a fluid communication may be created between the first needle 240 and the second needle 250 with the primary medicament of the first cartridge and the secondary medicament of the second cartridge, respectively.

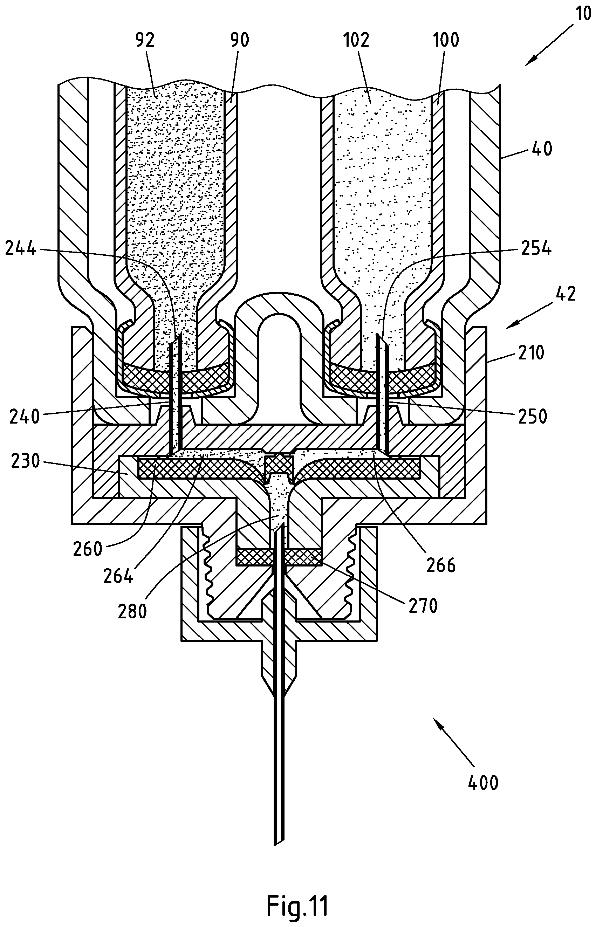

Fig. 11 illustrates the dispense interface 200 after it has been mounted onto the distal end 42 of the cartridge holder 40 of the drug delivery device 10 illustrated in Fig. 1. A double ended needle 400 is also mounted to the distal end of this interface. The cartridge holder 40 is illustrated as having a first cartridge containing a first medicament and a second cartridge containing a second medicament.

When the interface 200 is first mounted over the distal end of the cartridge holder 40, the proximal piercing end 244 of the first piercing needle 240 pierces the septum of the first cartridge 90 and thereby resides in fluid communication with the primary medicament 92 of the first cartridge 90. A distal end of the first piercing needle 240 will also be in fluid communication with a first fluid path groove 264 defined by the valve seal 260.

Similarly, the proximal piercing end 254 of the second piercing needle 250 pierces the septum of the second cartridge 100 and thereby resides in fluid communication with the secondary medicament 102 of the second cartridge 100. A distal end of this second piercing needle 250 will also be in fluid communication with a second fluid path groove 266 defined by the valve seal 260.

Fig. 11 illustrates a preferred arrangement of such a dispense interface 200 that is coupled to a distal end 15 of the main body 14 of drug delivery device 10. Preferably, such a dispense interface 200 is removably coupled to the cartridge holder 40 of the drug delivery device 10.

As illustrated in Fig. 11, the dispense interface 200 is coupled to the distal end of a cartridge housing 40. This cartridge holder 40 is illustrated as containing the first cartridge 90 containing the primary medicament 92 and the second cartridge 100 containing the secondary medicament 102. Once coupled to the cartridge housing 40, the dispense interface 200 essentially provides a mechanism for providing a fluid communication path from the first and second cartridges 90, 100 to the common holding chamber 280. This holding chamber 280 is illustrated as being in fluid communication with a dose dispenser. Here, as illustrated, this dose dispenser comprises the double ended needle assembly 400. As illustrated, the proximal end of the double ended needle assembly is in fluid communication with the chamber 280.

In one preferred arrangement, the dispense interface is configured so that it attaches to the main body in only one orientation, that is it is fitted only one way round. As such as illustrated in Fig. 11, once the dispense interface 200 is attached to the cartridge holder 40, the primary needle 240 can only be used for fluid communication with the primary medicament 92 of the first cartridge 90 and the interface 200 would be prevented from being reattached to the holder 40 so that the primary needle 240 could now be used for fluid communication with the secondary medicament 102 of the second cartridge 100. Such a one way around connecting mechanism may help to reduce potential cross contamination between the two medicaments 92 and 102.

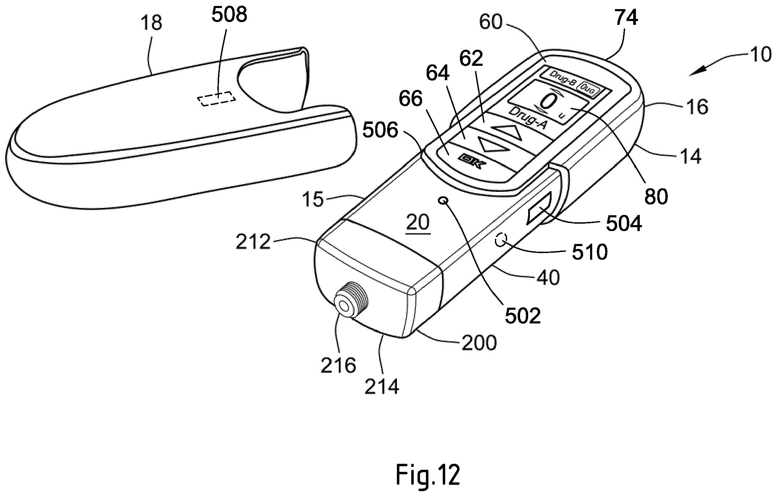

Figure 12 shows a perspective view of an alternative embodiment of the delivery device shown in Fig. 1. The delivery device shown in Fig. 12 differs from the delivery device of Fig. 1 in that the device further comprises a sensor 502 to detect whether end cap 18 is attached to the distal end 15 of the main body 14. Main body 14 here corresponds to the housing and distal end 15 acts as the cap connection part for removably attaching thereto cap 18. The delivery device shown in Fig. 12 furthermore comprises door opening buttons 504, 506 for opening cartridge retainers 50, 52 and a return button (not shown).

Sensor 502 may be reed or hall sensor and the cap 18 may comprise a corresponding magnet 504, which creates a magnetic field that is detected by sensor 502 when cap 18 is properly attached to the device. Magnet 504 may for example be embedded into the cap material as illustrated in Figure 12.

Cartridge holder 40 with cartridge retainers 50, 52 may be at least partially transparent so that the cartridges 90, 100 in the cartridge holder 40 may be identified from the outside. When the cap 18 is attached to the device, the cartridges are completely protected from light radiation. However, when cap 18 is detached from the device, light radiation may penetrate through the cartridge holder and cartridge retainer material and impinge on the cartridges 90, 100 and on the medicaments 92, 102 contained therein.

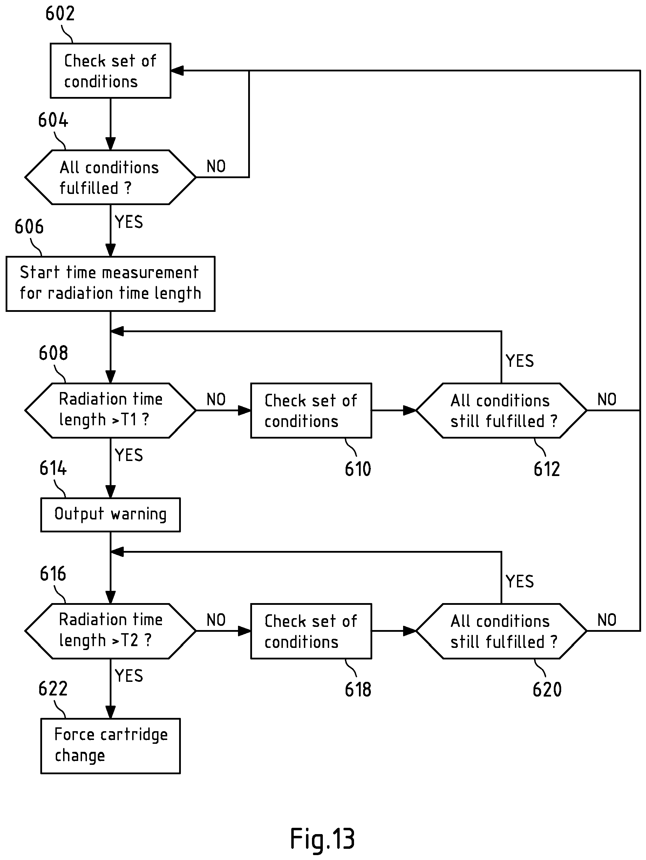

To prevent excessive light exposure of the drugs, the control unit of the device shown in Fig. 12 is configured to cause a control step to be performed when cap 18 is detached from the device for too long. To this end, the control unit may comprise a storage with commands the execution of which by the control unit causes a control sequence like for example the control sequence illustrated in Fig. 13 to be performed.

Figure 13 shows a flow diagram of an exemplary control sequence performed by the control unit of the device shown in Fig. 12 in order to reduce the risk of excessive light radiation on cartridges 90, 100 and on the medicaments 92, 102 contained therein and therewith to reduce the risk of medicaments degeneration.

In a first step 602, the control unit checks whether a set of conditions is fulfilled. The set of conditions may for example comprise the two conditions that a) cap 18 is not attached to the distal end 15 of the main body 14 and that b) no user activatable button, such as in particular buttons 62, 64, 66, 74, 504, 506 or the return button (not shown), is operated by the user. As long as at least one condition of the set of conditions is not fulfilled (step 604) the control unit loops back to step 602. For example, step 602 may be performed continuously or periodically in intervals of, for example, 1 s during this loop.

Once all conditions of the set of conditions are fulfilled, the control unit starts a time measurement in step 606 to measure the radiation time length and then assesses whether the radiation time length exceeds a predefined first threshold time length T1 which may for example be 90 seconds.

As long as the radiation time length is smaller than T1, control unit continuously checks whether still all conditions of the set of conditions are fulfilled (step 610) and returns to step 602 if this is not the case (step 612).

Once the radiation time length exceeds T1, the control unit in step 614 causes output of a warning message to the user that the cap has to be attached to the device, for example by outputting (here: displaying) a message on digital display 80, a sound, a blinking dose button and/or the like, and then continues to assess whether the radiation time length exceeds a predefined second threshold time length T2, which may be 360 seconds for example (step 616) and whether all conditions of the set of conditions are still fulfilled (steps 618, 620). If the latter is no more the case, the control unit returns to step 602.

Once the radiation time length exceeds T2, control unit continues with step 622 and forces a cartridge, in particular both cartridges 90, 100 to be replaced. For example step 622 may comprise setting the device into a status, in which injection of a fluid is only allowed after replacement of the cartridges 90, 100.

With the control sequence described above, the control unit performs a two-level escalation for the case that cap 18 is not attached to the device after use: After a first time length T1 the user is warned of a looming medicament degeneration if the cap is not replaced and after a second time length T2, when there is a considerable risk that the medicaments already have started to degenerate, further medicament ejection is prohibited.

Alternatively or in addition to measuring the radiation time length, the control unit may also be configured to determine the actual amount of radiation a cartridge within one of the cartridge retainers is exposed to. To this end, the delivery device shown in Fig. 12 may comprise a radiation sensor 510 such as a light sensor, in particular a UV sensor, and the control unit may be configured to determine the amount of radiation by integrating the radiation sensor signal over a time period and to cause a control step (such as, for example, steps 614 or 622) to be performed once the amount of radiation exceeds a predefined threshold amount of radiation.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta¬decanoyl) human insulin.

Exendin-4 for example means Exendin-4(1-39), a peptide of the sequence H His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;
or an Exendin-4 derivative of the sequence
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (∼150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (CH) and the variable region (VH). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCI or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

## Claims

1. An apparatus for ejecting a fluid,
- having a housing comprising a cartridge retainer (50, 52) configured to hold a fluid containing cartridge (90, 100) and further comprising a cap connection part for removably attaching thereto a cap (18), wherein the housing is configured such that a cartridge (90, 100) in the cartridge retainer (50, 52) is protected from a radiation by the cap when the cap (18) is attached to the cap connection part,
- **characterized in that** the apparatus has a sensor (502) configured to determine whether the cap (18) is attached to the connection part, and the apparatus further has a control unit configured to measure a quantity being a measure for the radiation load on a cartridge in the cartridge retainer if the cap is not attached to the connection part, and
- wherein the control unit is further configured to cause a control step to be performed once the quantity exceeds a predefined threshold value.

2. The apparatus according to claim 1,
- wherein the control unit is configured to measure a radiation time length, the radiation time length being the length of a continuous time period during which a set of conditions is fulfilled, the set of conditions at least comprising the condition that the cap (18) is not attached to the connection part, and
- wherein the control unit is configured to cause a control step to be performed once the radiation time length exceeds a predefined threshold time length (T1, T2).

3. The apparatus according to claim 1 or 2,
- wherein the apparatus comprises a radiation sensor (510), the radiation sensor being configured to measure the intensity of a radiation, preferably of light, in particular of UV light,
- wherein the control unit is configured to measure the amount of radiation employing the radiation sensor, and
- wherein the control unit is configured to cause a control step to be performed once the amount of radiation exceeds a predefined threshold amount of radiation.

4. The apparatus according to one of claims 1 to 3, wherein the apparatus further comprises a user interface for outputting messages to a user and wherein the control step comprises outputting of a warning message by means of the user interface.

5. The apparatus according to one of claims 1 to 4, wherein the control step comprises setting the apparatus into a status, in which ejection of a fluid is only allowed after replacement of the cartridge (90, 100) in the cartridge retainer (50, 52).

6. The apparatus according to one of claims 2 to 5, wherein the control unit is configured to cause a first control step to be performed once the radiation time length exceeds a predefined first threshold time length (T1) and wherein the control unit is configured to cause a second control step to be performed once the radiation time length exceeds a predefined second threshold time length (T2), the second threshold time length being longer than the first threshold time length and the first control step being different from the second control step.

7. The apparatus according to claim 6, wherein the apparatus comprises a user interface for outputting messages to a user, wherein the first control step comprises outputting of a warning message by means of the user interface, and wherein the second control step comprises setting the apparatus into a status, in which ejection of a fluid is only allowed after replacement of the cartridge (90, 100) in the cartridge retainer (50, 52).

8. The apparatus according to one of claims 2 to 7, wherein the set of conditions further comprises the condition that no user activatable button (62, 64, 66, 74, 504, 506) of the apparatus is operated.

9. The apparatus according to one of claims 2 to 8, wherein the set of conditions further comprises the condition that the apparatus is in a stand-by status.

10. The apparatus according to one of claims 1 to 9, wherein the sensor (502) is a reed sensor or a hall sensor.

11. The apparatus according to one of claims 1 to 10, wherein the apparatus is a medical device, in particular a medicament injection device.

12. A system, comprising an apparatus according to one of claims 1 to 11 and a cap (18) configured to be removably attached to the connection part of the apparatus.

13. The system according to claim 12, wherein the material, the wall thickness and/or the color of the cap (18) is selected such that the light transmittance of the cap is less than 1 %.

14. A method for controlling an apparatus according to any one of claims 1 to 11 or a system according to claim 12 or 13 **characterised in that** the method comprises the following steps being performed by the control unit:
- measuring a quantity being a measure for the radiation load on a cartridge in the cartridge retainer; and
- performing a control step once the quantity exceeds a predefined threshold value.

## Patentansprüche

1. Einrichtung zum Abgeben eines Fluids,
- mit einem Gehäuse, das eine Kartuschenhalterung (50, 52) umfasst, die zum Halten einer Fluid enthaltenden Kartusche (90, 100) konfiguriert ist, und das ferner einen Kappenverbindungsteil zum entfernbaren Anbringen einer Kappe (18) daran umfasst, wobei das Gehäuse so konfiguriert ist, dass eine Kartusche (90, 100) in der Kartuschenhalterung (50, 52) durch die Kappe vor einer Strahlung geschützt wird, wenn die Kappe (18) an dem Kappenverbindungsteil angebracht ist,
- **dadurch gekennzeichnet, dass** die Einrichtung einen Sensor (502) aufweist, der zum Bestimmen konfiguriert ist, ob die Kappe (18) an dem Verbindungsteil angebracht ist, und dass die Einrichtung ferner eine Steuereinheit aufweist, die zum Messen einer Quantität konfiguriert ist, die ein Maß für die Strahlungsbelastung auf eine Kartusche in der Kartuschenhalterung ist, falls die Kappe nicht an dem Verbindungsteil angebracht ist, und
- wobei die Steuereinheit ferner zum Bewirken konfiguriert ist, dass ein Steuerschritt durchgeführt wird, sobald die Quantität einen vordefinierten Schwellenwert überschreitet.

2. Einrichtung nach Anspruch 1,
- wobei die Steuereinheit zum Messen einer Strahlungszeitlänge konfiguriert ist, wobei die Strahlungszeitlänge die Länge einer kontinuierlichen Zeitperiode ist, während der ein Satz von Bedingungen erfüllt wird, wobei der Satz von Bedingungen wenigstens die Bedingung umfasst, dass die Kappe (18) nicht an dem Verbindungsteil angebracht ist, und
- wobei die Steuereinheit zum Bewirken konfiguriert ist, dass ein Steuerschritt durchgeführt wird, sobald die Strahlungszeitlänge eine vordefinierte Schwellenzeitlänge (T1, T2) überschreitet.

3. Einrichtung nach Anspruch 1 oder 2,
- wobei die Einrichtung einen Strahlungssensor (510) umfasst, wobei der Strahlungssensor zum Messen der Intensität einer Strahlung, bevorzugt von Licht, insbesondere von UV-Licht, konfiguriert ist,
- wobei die Steuereinheit zum Messen der Menge an Strahlung unter Nutzung des Strahlungssensors konfiguriert ist, und
- wobei die Steuereinheit zum Bewirken konfiguriert ist, dass ein Steuerschritt durchgeführt wird, sobald die Menge an Strahlung eine vordefinierte Schwellenmenge an Strahlung überschreitet.

4. Einrichtung nach einem der Ansprüche 1 bis 3, wobei die Einrichtung ferner eine Benutzerschnittstelle zum Ausgeben von Nachrichten an einen Benutzer umfasst und wobei der Steuerschritt Ausgeben einer Warnungsnachricht mittels der Benutzerschnittstelle umfasst.

5. Einrichtung nach einem der Ansprüche 1 bis 4, wobei der Steuerschritt Setzen der Einrichtung in einen Zustand umfasst, in dem eine Abgabe eines Fluids nur nach einem Austausch der Kartusche (90, 100) in der Kartuschenhalterung (50, 52) erlaubt ist.

6. Einrichtung nach einem der Ansprüche 2 bis 5, wobei die Steuereinheit zum Bewirken konfiguriert ist, dass ein erster Steuerschritt durchgeführt wird, sobald die Strahlungszeitlänge eine vordefinierte erste Schwellenzeitlänge (T1) überschreitet, und wobei die Steuereinheit zum Bewirken konfiguriert ist, dass ein zweiter Steuerschritt durchgeführt wird, sobald die Strahlungszeitlänge eine vordefinierte zweite Schwellenzeitlänge (T2) überschreitet, wobei die zweite Schwellenzeitlänge länger als die erste Schwellenzeitlänge ist und der erste Steuerschritt verschieden von dem zweiten Steuerschritt ist.

7. Einrichtung nach Anspruch 6, wobei die Einrichtung eine Benutzerschnittstelle zum Ausgeben von Nachrichten an einen Benutzer umfasst, wobei der erste Steuerschritt Ausgeben einer Warnungsnachricht mittels der Benutzerschnittstelle umfasst und wobei der zweite Steuerschritt Setzen der Einrichtung in einen Zustand umfasst, in dem eine Abgabe eines Fluids nur nach einem Austausch der Kartusche (90, 100) in der Kartuschenhalterung (50, 52) erlaubt ist.

8. Einrichtung nach einem der Ansprüche 2 bis 7, wobei der Satz von Bedingungen ferner die Bedingung umfasst, dass keine benutzeraktivierbare Taste (62, 64, 66, 74, 504, 506) der Einrichtung bedient wird.

9. Einrichtung nach einem der Ansprüche 2 bis 8, wobei der Satz von Bedingungen ferner die Bedingung umfasst, dass sich die Einrichtung in einem Stand-By-Zustand befindet.

10. Einrichtung nach einem der Ansprüche 1 bis 9, wobei der Sensor (502) ein Reed-Sensor oder ein Hall-Sensor ist.

11. Einrichtung nach einem der Ansprüche 1 bis 10, wobei die Einrichtung eine medizinische Vorrichtung, insbesondere eine Medikamenteninjektionsvorrichtung ist.

12. System, das eine Einrichtung nach einem der Ansprüche 1 bis 11 und eine Kappe (18) umfasst, die dazu konfiguriert ist, entfernbar an dem Verbindungsteil der Einrichtung angebracht zu werden.

13. System nach Anspruch 12, wobei das Material, die Wanddicke und/oder die Farbe der Kappe (18) so gewählt werden, dass der Lichttransmissionsgrad der Kappe geringer als 1 % ist.

14. Verfahren zum Steuern einer Einrichtung nach einem der Ansprüche 1 bis 11 oder eines Systems nach Anspruch 12 oder 13, **dadurch gekennzeichnet, dass** das Verfahren die folgenden Schritte umfasst, die durch die Steuereinheit durchgeführt werden:
- Messen einer Quantität, die ein Maß für die Strahlungsbelastung auf eine Kartusche in der Kartuschenhalterung ist; und
- Durchführen eines Steuerschrittes, sobald die Quantität einen vordefinierten Schwellenwert überschreitet.

## Revendications

1. Appareil destiné à éjecter un fluide,
- ayant un boîtier comprenant un porte-cartouche (50, 52) configuré pour tenir une cartouche contenant un fluide (90, 100) et comprenant en outre une partie de raccordement de capuchon pour y attacher de façon amovible un capuchon (18), le boîtier étant configuré de telle sorte qu'une cartouche (90, 100) dans le porte-cartouche (50, 52) est protégée d'un rayonnement par le capuchon quand le capuchon (18) est attaché à la partie de raccordement de capuchon,
- **caractérisé en ce que** l'appareil a un capteur (502) configuré pour déterminer si le capuchon (18) est attaché à la partie de raccordement, et
l'appareil a en outre une unité de commande configurée pour mesurer une quantité qui est une mesure de la charge de rayonnement sur une cartouche dans le porte-cartouche si le capuchon n'est pas attaché à la partie de raccordement,
et
- dans lequel l'unité de commande est également configurée pour provoquer l'exécution d'une étape de commande une fois que la quantité dépasse une valeur seuil prédéfinie.

2. Appareil selon la revendication 1,
- dans lequel l'unité de commande est configurée pour mesurer une durée de rayonnement, la durée de rayonnement étant la durée d'un laps de temps continu pendant lequel un ensemble de conditions est rempli, l'ensemble de conditions comprenant au moins la condition que le capuchon (18) n'est pas attaché à la partie de raccordement, et
- dans lequel l'unité de commande est configurée pour provoquer l'exécution d'une étape de commande une fois que la durée de rayonnement dépasse une durée seuil prédéfinie (T1, T2).

3. Appareil selon la revendication 1 ou 2,
- l'appareil comprenant un capteur de rayonnement (510), le capteur de rayonnement étant configuré pour mesurer l'intensité d'un rayonnement, de préférence de lumière, en particulier de lumière UV,
- dans lequel l'unité de commande est configurée pour mesurer la quantité de rayonnement en employant le capteur de rayonnement, et
- dans lequel l'unité de commande est configurée pour provoquer l'exécution d'une étape de commande une fois que la quantité de rayonnement dépasse une quantité de rayonnement seuil prédéfinie.

4. Appareil selon une des revendications 1 à 3,
l'appareil comprenant en outre une interface utilisateur destinée à délivrer des messages à un utilisateur et l'étape de commande comprenant la délivrance d'un message d'avertissement au moyen de l'interface utilisateur.

5. Appareil selon une des revendications 1 à 4,
dans lequel l'étape de commande comprend le réglage de l'appareil dans un état dans lequel l'éjection d'un fluide est autorisée uniquement après le remplacement de la cartouche (90, 100) dans le porte-cartouche (50, 52).

6. Appareil selon une des revendications 2 à 5,
dans lequel l'unité de commande est configurée pour provoquer l'exécution d'une première étape de commande une fois que la durée de rayonnement dépasse une première durée seuil prédéfinie (T1) et dans lequel l'unité de commande est configurée pour provoquer l'exécution d'une deuxième étape de commande une fois que la durée de rayonnement dépasse une deuxième durée seuil prédéfinie (T2), la deuxième durée seuil étant plus longue que la première durée seuil et la première étape de commande étant différente de la deuxième étape de commande.

7. Appareil selon la revendication 6,
l'appareil comprenant une interface utilisateur destinée à délivrer des messages à un utilisateur, la première étape de commande comprenant la délivrance d'un message d'avertissement au moyen de l'interface utilisateur, et la deuxième étape de commande comprenant le réglage de l'appareil dans un état dans lequel l'éjection d'un fluide est autorisée uniquement après le remplacement de la cartouche (90, 100) dans le porte-cartouche (50, 52).

8. Appareil selon une des revendications 2 à 7,
dans lequel l'ensemble de conditions comprend en outre la condition qu'aucun bouton activable par l'utilisateur (62, 64, 66, 74, 504, 506) de l'appareil n'est actionné.

9. Appareil selon une des revendications 2 à 8,
dans lequel l'ensemble de conditions comprend en outre la condition que l'appareil est dans un état d'attente.

10. Appareil selon une des revendications 1 à 9,
dans lequel le capteur (502) est un capteur Reed ou un capteur à effet Hall.

11. Appareil selon une des revendications 1 à 10,
l'appareil étant un dispositif médical, en particulier un dispositif d'injection de médicament.

12. Système, comprenant un appareil selon une des revendications 1 à 11 et un capuchon (18) configuré pour être attaché de façon amovible à la partie de raccordement de l'appareil.

13. Système selon la revendication 12,
dans lequel le matériau, l'épaisseur de paroi et/ou la couleur du capuchon (18) sont choisi de telle sorte que la transmittance lumineuse du capuchon est inférieure à 1 %.

14. Procédé de commande d'un appareil selon l'une quelconque des revendications 1 à 11 ou d'un système selon la revendication 12 ou 13, **caractérisé en ce que** le procédé comprend les étapes suivantes qui sont effectuées par l'unité de commande :
- mesurer une quantité qui est une mesure de la charge de rayonnement sur une cartouche dans le porte-cartouche ; et
- exécuter une étape de commande une fois que la quantité dépasse une valeur seuil prédéfinie.
